# EUROPEAN PATENT APPLICATION

(11) **EP 1 505 153 A1**
(43) Date of publication of application: **09.02.2005**
(21) Application number: 03731726.0
(22) Date of filing: 24.01.2003
(51) Int. Cl.: C12N 15/11, C12N 15/86, A61K 39/125, A61K 39/42

(54) **NUCLEIC ACID SEQUENCE COMPRISING THE RNA PACKAGING SIGNAL OF A GROUP 1 CORONAVIRUS AND THE APPLICATIONS THEREOF**

(30) Priority: 24.01.2002 ES 200200158
(71) Applicant: CONSEJO SUPERIOR DE INVESTIGACIONES CIENTIFICAS, 28006 Madrid (ES)
(72) Inventor: Enjuanes Sanchez, Luis, 28049 Madrid (ES); Escors Murugarren, David, 28049 Madrid (ES); Ortego Alonso, Javier, 28049 Madrid (ES)
(74) Representative: UEXKÜLL & STOLBERG
(86) International application number: PCT/ES2003/000038
(87) International publication number: WO 2003/062424

(57) **Abstract**

The nucleic acid sequence includes the RNA encapsidation signal of a group 1 coronavirus such as porcine transmissible gastroenteritis virus (TGEV) situated between nucleotides 100 and 649 of the genome of said coronavirus. This sequence may be used to construct viral vectors that are useful in basic and applied research, for example, in the development of systems for expression of products of interest, vaccine vectors, and gene therapy.

## Description

### Field of the invention

This invention pertains to a nucleic acid sequence that includes the encapsidation signal (ES) of the RNA of a group 1 coronavirus and its use in the construction of vectors useful in the fields of virology and health.

### Background of the invention

Viruses with a RNA genome (RNA viruses) are potential sources of viral vectors useful in vaccines or gene therapy. This type of application requires that the viral vectors be biosafe. Although the viral vectors derived from RNA viruses are relatively safe, we cannot rule out the possibility that they may recover their virulence by recombination with circulating viruses, which considerably limits their possibilities of use in both basic and applied research. For this reason, there is a need to develop viral vectors derived from RNA viruses with a high level of biosafety.

Among the viral vectors derived from RNA viruses that are potentially useful in human or animal health are viral vectors derived from coronaviruses (Spanish patent application P9902673). Coronaviruses are ssRNA(+) viruses that present the largest known genome for an RNA virus, with a length of from 27.6 to 31 kilobases (kb). The genome of coronaviruses includes a sequence of nucleotides involved in the encapsidation or the genome in the virions, which we will call the encapsidation sequence or signal (ES). The absence of alteration of this ES causes reduced or zero encapsidation of the genome in the virions, which may be valuable for some applications.

Although an ES of 61 nt has been characterized in the genome of the coronavirus MHV (murine hepatitis virus) located at ORF 1b, 1.4 kb from the 3'-terminal of gene I, and a homologous signal in the genome of bovine coronavirus (BCoV) (Cologna and Hogue, 2000; Fosmire et al., 1992; Woo et al., 1997), there is a need to localize and characterize said ESs in other coronaviruses that are useful in various applications in both basic and biomedical research, for example the coronavirus TGEV (porcine transmissible gastroenteritis virus). Localization and characterization of this ES in other coronaviruses will make it possible to construct new viral vectors derived from these coronaviruses with a higher level of biosafety, and by using this model to expand the arsenal of tools available for basic and applied research.

### Summary of the invention

We have now localized the region of the TGEV virus genome that includes the ES of this virus, which has permitted the development of new vectors characterized by their high degree of biosafety. The new vector provided by this invention can be used in basic or applied research, for example, to obtain products of interest (proteins, enzymes, antibodies, etc.), as a vaccination vector or in human or animal gene therapy. The advantage of this new vector, in addition to the capacity to infect enteric and respiratory mucosa, i.e., the sites most suitable for induction of secretory immunity and from which its tropism can be controlled by modification of the S (spike) gene, is that it can be constructed with a high degree of biosafety.

### Brief description of the figures

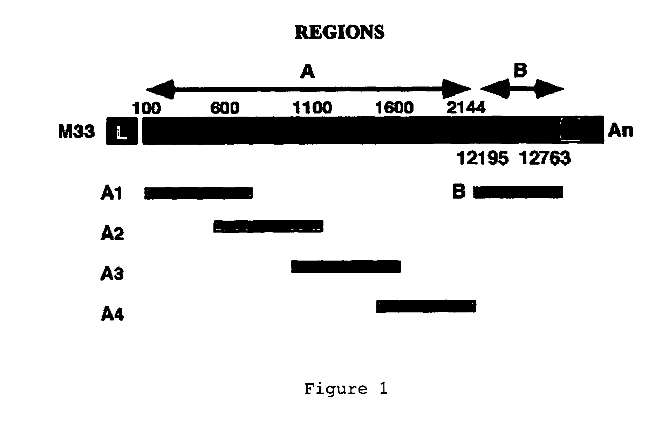

Figure 1 illustrates the selection of the regions that potentially contain the TGEV encapsidation signal. The bar on the upper part of the figure represents the M33 minigenome. Over the bar of the minigenome are indicated the two regions of the 5'-terminal of the M33 minigenome designated by double-headed arrows (regions A and B). Under zones A and B, the subregions under investigation are shown in schematic form. The numbers over and under the bar indicate the positions of the TGEV genome.

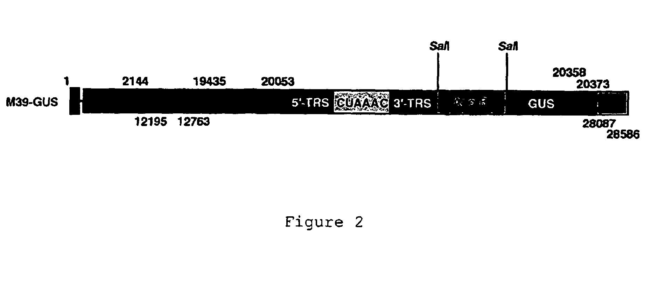

Figure 2 is a diagram illustrating the cloning of regions potentially involved in encapsidation in the M39-GUS minigenome. This diagram represents the M39-GUS minigenome with the inserts corresponding to the regions defined in Figure 1 (Ai or B) cloned under control of the TRS of the minigenome. The TRS sequence, the 5'CUAAAC-3' conserved sequence, the insert (Ai or B) and the gus gene are shown in blue. The figure indicates the position of the SalI restriction sequences which flank the cloned inserts in the minigenome. The numbers indicate the positions of TGEV in the genome.

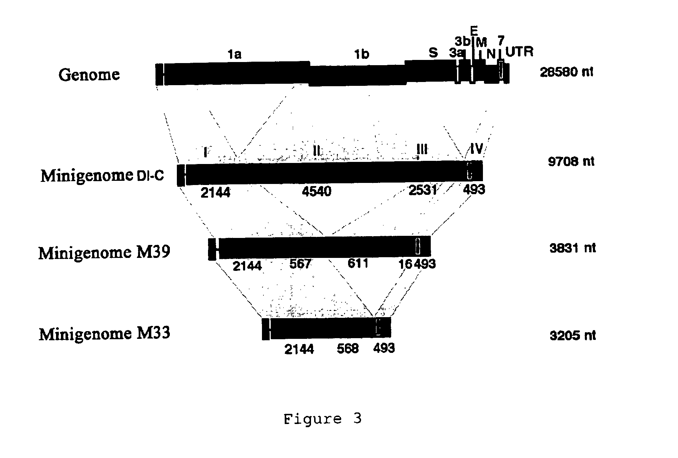

Figure 3 shows the structure of the DI-C RNA and of the M39 and M33 minigenomes, DI-C deletion mutants. The DI-C RNA is formed by four discontinuous regions of the TGEV genome (I, n, m, and IV). These regions include 2.1 kb of the 5'-terminal of the genome, the almost complete ORF 1b including the zone of overlap between ORFs 1a and 1b, the 5'-terminal of the S gene, the incomplete ORF 7 and the UTR of the 3'-terminal. From the DI-C genome we obtained, by deletions, the M39 and M33 minigenomes. The letters and numbers on the genome box indicate the viral genes. Below the bars representing the DI-C, M39, and M33 minigenomes, the length of the discontinuous regions is shown in nucleotides. The total length of the RNAs is shown on the right of the genome and minigenomes. The region identified as 2144 represents the genome sequence from position 1 to 2,144 of TGEV. The broad clear bar of the M33 minigenome represents the sequence between positions 12,195 and 12,762. The narrow clear and dark bars situated to the extreme right represent the sequence between positions 28,087 and 28,586.

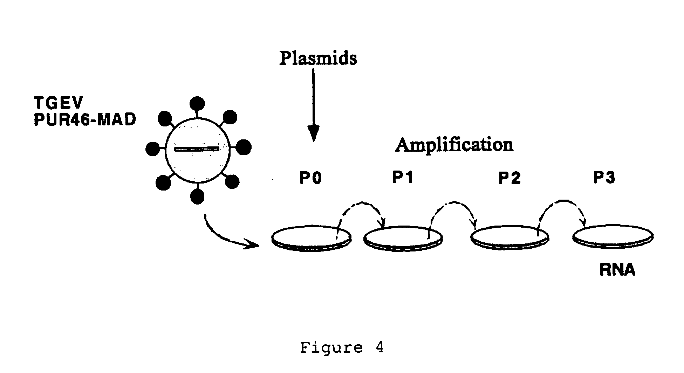

Figure 4 illustrates the recovery and amplification of the M39-GUS-A1, -A2, -A3, -A4, and 8 [sic; -B] minigenomes that are generated, and it represents the organization of the experimental procedure used, starting with the ST cells transfected with plasmids and infected with TGEV (PO [sic; passage zero, P0]). To amplify the minigenomes, the virus generated was passaged three consecutive times into confluent ST cells (PI, P2, and P3). In the third passage, the viral DNA was analyzed in the cell and in the purified virions.

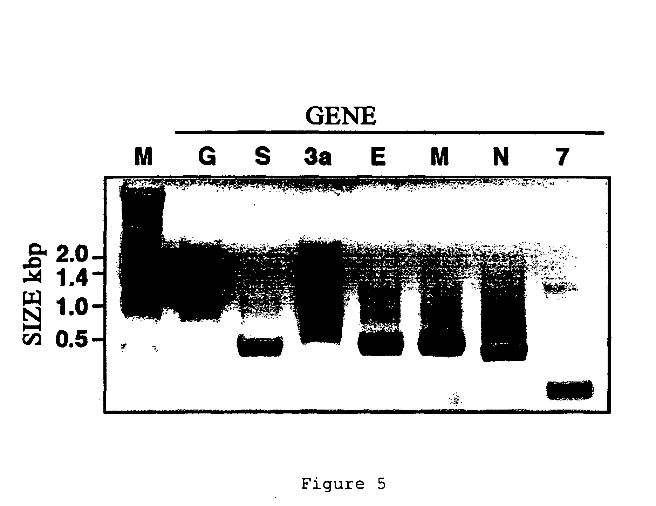

Figure 5 illustrates the detection of the subgenomic RNAs of TGEV by means of RT-PCR in infected cells, and shows the agarose gel electrophoresis of the RT-PCR reaction products that are specific for detection of each of the subgenomic RNAs of the virus, indicated in the upper part of the gel. The minority bands correspond to unidentified RT-PCR reaction products, and probably correspond to nonspecific hybridizations.

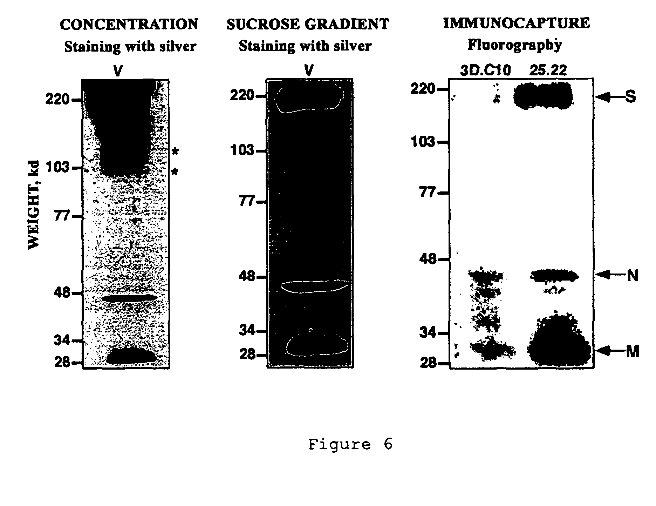

Figure 6 illustrates the analysis of the preparations of purified TGEV by three distinct procedures: a) analysis by SDS-PAGE electrophoresis and using silver nitrate to stain the TGEV concentrated by centrifugation through a 31 % sucrose cushion (gel on the left) or b) by centrifugation through a continuous sucrose gradient (gel in the center), or c) analysis by SDS-PAGE electrophoresis and fluorography of the TGEV that has been radiolabeled and purified by immunocapture (gel on the right). The arrows indicate the positions of the major * structural proteins, contaminating proteins.

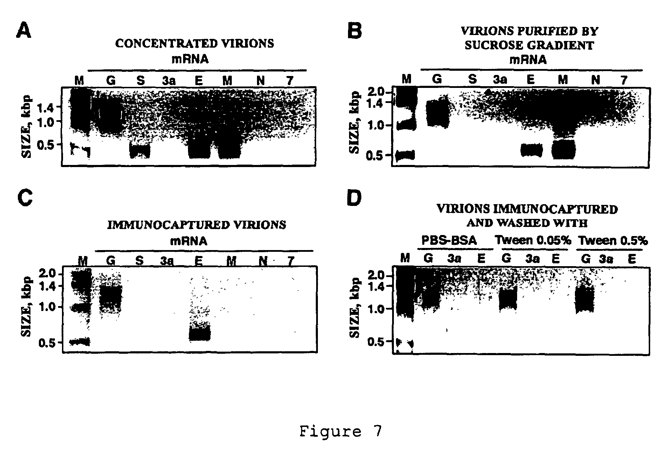

Figure 7 illustrates the detection of the mRNAs of the TGEV by RT-PCR in purified virions and shows the agarose gel electrophoresis of the RT-PCR reaction products obtained by detection of the mRNAs indicated in the upper part of the gels, in concentrated virus (A), virus purified by sucrose gradient (B), virus purified by immunocapture (C), and virus purified by immunocapture with 15 washings with a PES-ESA [sic; PBS-BSA] buffer solution alone and including the concentrations of the Tween-20 nonionic detergent indicated (D).

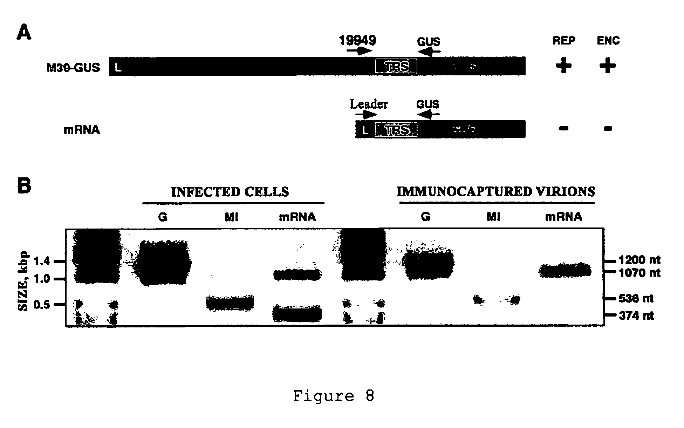

Figure 8 illustrates the detection of the genome, M39-GUS minigenome and mRNA-GUS. in ST cells infected with a TGEV inoculum that contained the M39-GUS minigenome, and in purified virions. Figure 8A shows the organization of the M39-GUS minigenome (top bar) and of the mRNA that is coded by it (bottom bar), detected by RT-PCR with the oligonucleotides used indicated over each bar. The leader sequence is shown in the 5'-terminals (L). On the right is an indication of whether the RNAs replicate (REP+) and encapsidate (ENC+). Figure 8B shows the result of the agarose gel electrophoresis of the products of the specific RT-PCR reactions for detection of the genome (G), minigenome (Mi), and mRNA-GUS (mRNA). The sizes of the products are shown to the right of the gel.

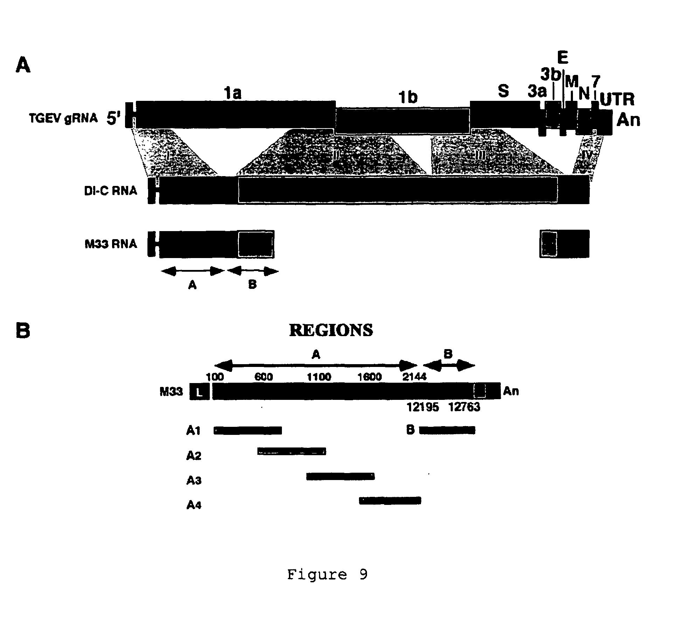

Figure 9 shows in schematic form the sequences potentially involved in the efficient recovery of the M33 minigenome. The organization shown in Figure 9A represents the genomic RNA of TGEV and the DI-C RNA generated from it. The structure of the M33 minigenome is compared to that of the RNA of the DI-C minigenome. A is the region of the 5'-terminal of the virus genome; B is the region proceeding from the overlapping sequence between ORFs la and 1ab. Each of the regions of the DI-C minigenome is shaded, indicating that it originates in the genome of the virus. Figure 1B shows the M33 minigenome in schematic form with regions A and B indicated. The subdivisions of each region are shown schematically as bars. The numbers indicate positions of the genome.

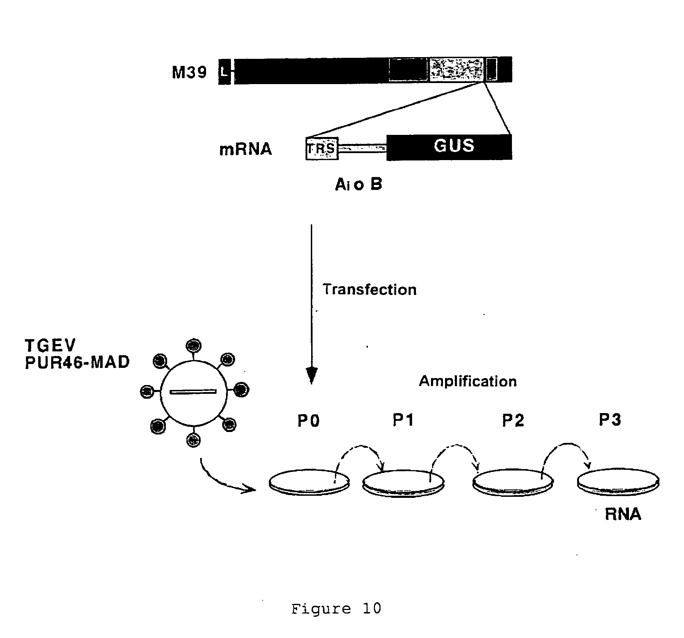

Figure 10 shows schematically the insertion of the sequences of 5'-terminal of the minigenome M33 in messenger mRNAs derived from the minigenome M39-GUS. The top diagram represents the minigenome M39-GUS in which there is inserted an expression module composed of an optimized IRS [sic; TRS], the inserted sequences (Ai or B), and the gus gene. The bottom diagram represents the transfection of the constructs generated, their recovery and amplification in successive stages in cells infected with the TGEV (PO [sic; P0] to P3). The RNA of the cells and the virions was purified in step 3.

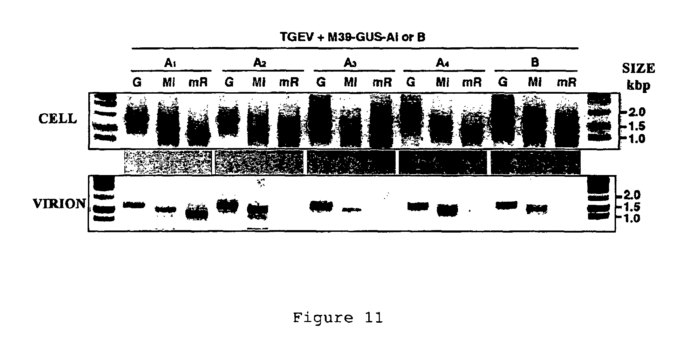

Figure 11 shows the results of the analysis by RT-PCR of the RNAs amplified in infected cells in the presence of minigenomes that include the sequences A1, A2, A3, A4, and B. In the gel are shown the reaction products of RT-PCR specific for detection of the genome (O [sic; G]), minigenome (Mi), and mRNAs (mR) in the infected cells. In the lower gel are shown the products of the reactions of specific RT-PCRs detected in purified virions, corresponding to the analyses shown in the upper gel. Amplification by PCR of the mRNAs and minigenomes was effected with the oligonucleotide OUS 297, which was not hybridized with the virus genome.

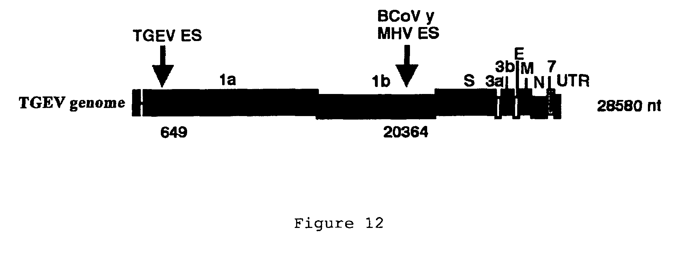

Figure 12 illustrates the location of the encapsidation signal in the genome of TGEV. The genome of TGEV, including the various ORFs of the virus, is represented schematically. The position of the encapsidation signals of the coronaviruses TGEV, MHV, and BCoV are indicated by the arrow. The approximate position of the encapsidation signals in the virus genome is shown under the genome. ES, encapsidation signal.

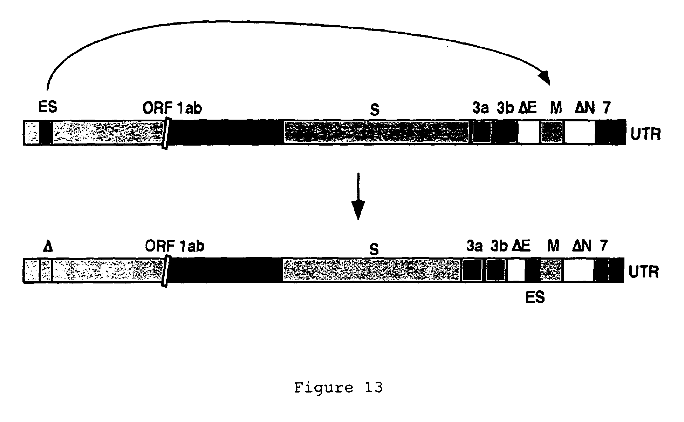

Figure 13 illustrates schematically the relocation of the encapsidation signal in the genome of TGEV. The top diagram shows the genome of TGEV with the ORFs E and N deleted (ΔE and ΔN). The various ORFs of the virus and the encapsidation signal (ES) are represented. The arrow indicates the relocation of the ES from 5'-terminal to 3'-terminal, resulting in a new genome with an encapsidation signal located in the position of the deleted ORF E (lower diagram). UTR, untranslatable region.

### Detailed description of the invention

In one aspect, the invention provides an isolated sequence of nucleic acid, called herein the nucleic acid sequence of the invention, selected from:
a) a sequence of nucleotides that includes from nucleotide 100 to nucleotide 649 of the genome of a group 1 coronavirus, said sequence of nucleotides including the encapsidation sequence (ES) of said coronavirus;
b) a nucleic acid sequence analogous to the sequence defined in a) which contains the encapsidation sequence (ES) of a group 1 coronavirus;
c) a nucleic acid sequence complementary to either of said sequences a) or b); and
d) a sequence of a nucleic acid with a secondary structure similar to that of an encapsidation sequence (ES) of a group 1 coronavirus, independently of the nucleotide sequence.

The term "nucleic acid" as used in this description includes any molecule of DNA or RNA.

Likewise, in the sense used in this invention, the term "group I coronavirus" includes porcine viruses such as TGEV and porcine epidemic diarrhea virus (PEDV); canine viruses such as canine coronavirus (CCoV); feline viruses such as feline infectious peritonitis virus (FIPV); human viruses (HCoV-229E), and other coronaviruses with sequences closely related to these.

As used in this description, the term 'analogous' includes any nucleic acid sequence that has a high percentage, 40% or more, of nucleotides that are identical or that present the same technical characteristic of the sequence defined in a), i.e., it contains the ES of a group 1 coronavirus. This analogous nucleic acid sequence may contain conservative or nonconservative nucleotide substitutions, or it may contain one or more additional nucleotides in any of its terminals, or it may present one or more deletions, provided that the ES of the group 1 coronavirus is maintained. In general, this analogous nucleic acid sequence defined in b) is substantially homologous to the nucleotide sequence defined in a). As used in this description, the expression "substantially homologous" means that the nucleotide-sequences in question have at least a 60% degree of identity, and preferably at least 80%, or most preferably at least 95%. In a particular embodiment, the nucleic acid sequence analogous to the sequence defined in a) [sequence b)] is a fragment of this sequence of nucleotides defined in a) which includes the ES of the group 1 coronavirus.

The invention also includes nucleic acid sequences complementary to those sequences identified as a) or b), as well as sequences of nucleic acids, particularly RNA, with secondary structures similar to those of, at least, one ES of a group 1 coronavirus, independently of the sequence of nucleotides. These secondary sequences were predicted using the M-fold software program (Zucker, 1989a and b) and their existence was proven by genetic, enzymatic, and chemical methods.

In one particular embodiment, the nucleic acid sequence of the invention includes, or is made up of, the sequence of nucleotides shown in SEQ. ID No.: 1. The sequence of nucleotides shown in SEQ. ID. No.: 1 corresponds to the sequence of nucleotides between nucleotide 100 and nucleotide 649 of the cDNA of the TGEV genome, specifically the PUR-46 MAD isolate, an isolated representative of TGEV, the complete sequence of which is described in Spanish patent application P9902673, and contains the ES of this virus. In another particular embodiment, the nucleic acid sequence of the invention includes a fragment of said SEQ. ID. No.: 1 which contains the ES of TGEV.

The location of the ES of TGEV between nucleotides 100 and 649 of the virus genome was surprising, among other reasons because the ES in the coronavirus MHV was situated at 1.4 kb from the 3'-terminal of the replicase gene (Fosmire et al., 1992), i.e., a zone that is almost 20,000 nt from the one described here for the ES in the genome of TGEV.

The nucleic acid sequence of this invention may proceed from any organism that contains it in natural form, for example, a group I coronavirus, or a host organism transformed with said sequence of nucleic acid or, in general, any organism containing it. Therefore, the nucleic acid sequence of the invention may be obtained by using conventional methods, for example, by techniques of isolation and identification of nucleic acids from any organism that contains it or from a host organism transformed with this nucleic acid sequence, by using probes or oligonucleotides prepared from the information on the nucleotide sequence provided by this invention.

One essential characteristic of the nucleic acid sequence of the invention lies in the fact that said sequence includes the ES of a group 1 coronavirus. The identification of the ES of the genome permits, among other things, the study of the coronavirus encapsidation mechanisms and the generation of new biosafe viral vectors for the design of new vaccines and for gene therapy, applicable to human and animal health. The location of the ES of the genome is of great practical as well as scientific importance, and it can be used, for example, in the development of vectors that are useful for vaccination or for gene therapy derived from said group 1 coronaviruses.

In another aspect, the invention provides a vector that comprises
a nucleic acid sequence (I) selected from (i) a nucleic acid sequence corresponding to the complete genome, or part of the genome, of a group 1 coronavirus, and (ii) a nucleic acid sequence of another virus that has a sequence identity equal to or greater than 60% of said sequence (i); from which there has been deleted at least one gene that is essential for the assembly of said virus, and
a nucleic acid sequence of the invention.

In a particular embodiment, said nucleic acid sequence of the invention has been relocated in a position different from the original (i.e., in a position different from the position in which the wild virus is usually found), for example, in a position adjacent to or immediately to the side of the deleted gene or one of the deleted genes, thereby giving rise to a biosafe vector, that is, one that could regenerate a virus competent at replication and propagation by recombination with another circulating virus with a frequency equal to or less than 10⁻¹⁰. Therefore, a vector such as the one offered by this invention presents a high level of biosafety since the frequency of regeneration of infectious virus from a recombination between the vector and a field virus is very low (See Example, section 3, "Discussion").

In a particular embodiment, the vector provided by this invention is a viral vector derived from the group 1 coronavirus, for example, TGEV, which includes a nucleic acid sequence corresponding to the genome of said group 1 coronavirus, or a fragment thereof, from which there has been deleted at least one gene that is essential for the assembly of said coronavirus and in which the nucleic acid sequence of the invention has been located in a position different from the original, for example, in a position adjacent to or immediately to the side of the deleted gene or one of the deleted genes.

In another aspect, the invention provides a nucleic acid construct that includes the nucleic acid sequence of the invention together with, optionally, a transcription promoter sequence. Said construct may occur in a RNA or in a cDNA copy of a plasmid or vector that possesses a replication origin, and is thereby potentially capable of replicating itself in an adequate cell. The assembly of the transcription regulatory elements, as well as the introduction of said construct into a plasmid, can be accomplished by conventional means (Sambrook et al., 1989). Said nucleic acid construct provided by this invention as well as the plasmids that code for it constitute additional objects of this invention.

From the vector provided by this invention, it is possible to transform appropriate cells and to recover the virions obtained, which contain the construct provided by this invention. Therefore, the invention also provides a method for producing a recombinant coronavirus that includes the introduction of a vector provided by this invention into a host cell, cultivation of said cell under conditions that permit the expression and replication of the viral vector, and the recovery of the virions obtained. The vector of the invention can be introduced into the host cell by conventional methods. Said host cells that contain the vector of the invention constitute an additional object of this invention.

In addition, the vector provided by this invention can be manipulated by conventional genetic engineering techniques to insert at least one heterologous nucleic acid sequence which codes for a specific activity under control of the transcription regulatory elements present in said vector. Therefore, in another particular embodiment, the vector provided by this invention also includes a heterologous nucleic acid under the control of the transcription-regulatory elements present in said vector to permit expression of said heterologous nucleic acid.

As used in this description, the term "heterologous" applied to a nucleic acid refers to a nucleic acid sequence that is not normally present in the vector used to introduce the heterologous nucleic acid into a host cell.

The heterologous nucleic acid that may contain the viral vector of the invention may be a gene, or a fragment thereof, that codes for a protein, a peptide, an epitope or any gene product of interest (for example, antibodies, enzymes, etc.). Heterologous nucleic acid may be inserted into the vector of the invention by conventional genetic engineering techniques in any appropriate region of the cDNA, for example, after the codon initiating the translation of a gene that codes for a protein of a group 1 coronavirus and in the reading phase with this gene.

The vector of the invention may express one or more activities. In the latter case, the viral vector will include as many heterologous nucleic acid sequences as activities to be expressed, preceded by one or more promoters, or a promoter and various ribosome recognition sites (IRES [internal ribosome entry sites]), or various promoters and a ribosome recognition site.

In another aspect, the invention provides a method for producing a product of interest that includes cultivating a host cell that contains a viral vector of the invention under conditions that permit the expression of the heterologous nucleic acid and recovery of the product of interest. Said host cells that contain the viral vector of the invention constitute an additional object of this invention.

The viral vector of the invention can be designed so that its species specificity and its tropism can be readily controlled. Because of these characteristics, a very interesting application of the viral vectors of the invention is its use in gene therapy as a vector for the gene of interest or as a vaccine vector to introduce immune responses to different pathogens.

The invention also provides vaccines that can induce protection in an animal against the infection caused by an infectious agent that include (i) at least one viral vector of the invention that expresses at least one antigen adequate to induce an immune response against said infectious agent, or an antibody that provides protection against said infectious agent, together with, optionally (ii) a pharmaceutically acceptable inactive ingredient.

The term "induce protection" as used in this description is understood as the immune response of the recipient body (the animal to be immunized) induced by the viral vector of the invention, through adequate mechanisms such as those induced by substances that potentiate cellular response (interleukins, interferons, etc.), cellular necrosis factors and similar substances that protect the animal against infections caused by infectious agents.

The term "animal" includes any animal of any species, preferably mammalian, including humans.

The term "infectious agent" as used in this description includes any viral, bacterial, fungal, parasitic, or other infectious agent that can infect an animal and cause disease.

In a particular embodiment, the vaccine provided by this invention includes at least one viral vector of the invention that expresses at least one antigen capable of inducing a systemic immune response and/or an immune response in mucosas against various infectious agents that propagate in the respiratory or enteric mucosa. The vectors of this invention are adequate to induce immunity in mucosas as well as lactogenic immunity, which is of particular interest in protecting newborns against intestinal infections.

In another particular embodiment, the vaccine provided by this invention also includes at least one viral vector of the invention that expresses at least one gene that codes for the heavy and light chains of an antibody of any isotype (for example, IgG₁, IgA, etc.) that provides protection against an infectious agent.

Species specificity can be controlled in such a way that the viral vector expresses the S protein of the envelope of a coronavirus that infects the desired species (human, canine, feline, porcine, etc.) and that can be recognized by the cellular receptors of the species in question.

The vaccines provided by this invention may be monovalent or multivalent depending on whether the viral vectors of the invention express one or more antigens capable of inducing an immune response against one or more infectious agents or one or more antibodies that provide protection against one or more infectious agents.

In a particular embodiment of this invention, monovalent vaccines are provided which can protect humans, pigs, dogs, and cats against various human, porcine, canine, and feline infectious agents, and tropism is controlled by expressing the S glycoprotein of the coronavirus with the desired species specificity..

Monovalent vaccines against human infectious agents may contain a vector that expresses an antigen selected from the group made up essentially of antigens of human pathogens, especially human infectious agents selected from those that cause infections of the enteric and/or respiratory mucosa, or whose route of entry is through said mucosa, for example, human coronavirus, human rhinovirus, human rotavirus, human enterovirus, influenza virus, and in general any infectious pathogen including viruses and bacteria that infect humans.

Monovalent vaccines against porcine infectious agents may contain a vector that expresses an antigen selected from the group made up essentially of antigens of the following porcine pathogens: *Actinobacillus pleuropnemoniae, Actinobacillus suis, Haemophilus parasuis*, porcine parvovirus, *Leptospira, Escherichia coli,* Erysipelohrix rhusiopathiae, *Pasterlla multocida, Bordetella bronchiseptica, Clostridium sp., Serpulina hyodysenteriae, Mycoplasma hyopneumoniae*, PEDV, porcine respiratory coronavirus, rotavirus, foot-and-mouth disease virus (FMDV), or against pathogens that cause the porcine respiratory and reproductive syndrome, Aujeszky's disease (pseudorabies), porcine influenza or transmissible gastroenteritis, and the etiologic agent of atrophic rhinitis and proliferative ileitis.

Monovalent vaccines against infectious canine agents may contain an expression vector that expresses an antigen selected from the group made up essentially of antigens of the following canine pathogens: canine herpesvirus, types 1 and 2 canine adenovirus, types 1 and 2 canine parvovirus, canine retrovirus, canine rotavirus, canine coronavirus, canine parainfluenza virus, canine influenza virus, distemper virus, rabies virus, and canine calcivirus.

Monovalent vaccines against feline infectious agents may contain an expression vector that expresses an antigen selected from the group made up essentially of antigens of the following feline pathogens: cat calcivirus, feline immunodeficiency virus, feline herpesvirus, feline panleukemia virus, feline retrovirus, feline rotavirus, feline coronavirus, infectious peritonitis virus of the cat, rabies virus, feline *Chlamydia psittaci*, and feline leukemia virus.

The vectors of the invention can express an antibody that provides protection against an infectious agent, for example, a porcine, canine, or feline infectious agent as cited above. In a particular embodiment, the vector expresses the recombinant monoclonal antibody identified as 6A.C3 which neutralizes TGEV, expressed with isotypes IgG₁ or IgA in that the constant part of the immunoglobulin is of porcine origin or neutralizing antibodies for human and porcine rotaviruses.

A diluent, such as physiological saline or other similar saline solutions can be used as an excipient. These vaccines may also contain an adjuvant such as those normally used in the formulation of vaccines - either aqueous, such as aluminum hydroxide, QuilA, suspensions of alumina gel and similar agents, or oily, based on mineral oils, glycerides, and fatty acid derivatives, and mixtures thereof.

These vaccines may also contain substances that potentiate the cellular response (PRC) i.e., substance that potentiate subpopulations of helper T-cells (Th₁ and Th₂) such as interleukin-1 (IL-1), IL-2, IL-4, IL-5, IL-6, IL-12, g [sic; γ]-IFN (gamma-interferon), cell necrosis factor and similar substances, which may theoretically promote cellular immunity in vaccinated animals. These PRC substances may be used in vaccine formulations with aqueous or oily additives. It is also possible to use another type of additives that modulate and immunostimulate the cellular response such as MDP (muramyl dipeptide), ISCOM (ImmunoStimulant Complex), or liposomes.

The invention provides multivalent vaccines capable of preventing and protecting animals from infections caused by various infectious agents. These multivalent vaccines can be produced from viral vectors of the invention in which there have been inserted the various sequences that code for the corresponding antigens in the same recombinant vector or by constructing independent recombinant vectors that will subsequently be mixed together for joint inoculation. Therefore, these multivalent vaccines include a viral vector that contains more than one sequence of heterologous nucleic acids that code for more than one antigen or, alternatively, different viral vectors that express each at least one distinct antigen.

Similarly, it is possible to prepare multivalent vaccines that includes multivalent vectors using sequences that code for antibodies that provide protection against infectious agents in place of sequences that code for the antigens.

In a particular embodiment of this invention, vaccines are provided which are capable of conferring immunity on people, pigs, dogs, and cats against various human, porcine, canine, and feline viruses, respectively. For this purpose, the viral vectors contained in the vaccine must express different antigens of the human, porcine, canine, or feline pathogens mentioned above.

The vaccines of this invention may be present in liquid or freeze-dried form, and may be prepared by suspending the recombinant systems in the inactive ingredient. If said systems were in freeze-dried form, the inactive ingredient itself could be the reconstituting agent.

Alternatively, the vaccines provided by this invention can be used in combination with other conventional vaccines, either forming part of them or as a diluent or freeze-dried fraction to be diluted with other vaccines, whether conventional or recombinant.

The vaccines provided by this invention can be administered to the animal by the oral, nasal, subcutaneous, intradermal, intraperitoneal, or intramuscular route, or by aerosol.

One characteristic of the vaccines provided by this invention is their high level of biosafety, resulting from the fact that we start with vectors derived from viral genomes from which a gene has been deleted, which is essential to assembly of the virus, and in which the ES has been relocated in a position adjacent to or to the side of the deleted gene, so that the risk of restoring the wild virus by recombination is virtually nonexistent.

The invention also provides a method for immunization of animals, in particularly humans, pigs, dogs, and cats, against one or more infectious agents simultaneously, which includes oral, nasal, subcutaneous, intradermal, intraperitoneal, or intramuscular administration, or administration by aerosol (or combinations thereof) of a vaccine that contains an immunologically effective quantity of a recombinant system provided by this invention.

In addition, the invention also provides a method for protecting newborn animals against infectious agents that infect these animals, consisting of oral, nasal, subcutaneous, intradermal, intraperitoneal, or intramuscular administration, or administration by aerosol (or combinations thereof) to the mothers before or during pregnancy, or to the offspring, a vaccine such as those provided by this invention.

The invention is illustrated by the following example, which describes the identification of a region of the TGEV genome that contains the ES of said coronavirus. This example should not be considered as limiting the scope of the invention but only as an illustration of it.

### Examples

For the embodiment of Example 1, described in detail below, we generally used the Materials and Methods described below.

### Materials and Methods

### 1. Eukaryotic cells

### 1.1. Cell lines

The cells used for the growth, titration, and purification of the TGEV were ST (swine testicle) cells, a cell line obtained from epithelial cells of fetal pig testicles (McClurkin and Norman, 1966). These cells were obtained from L. Kemeny (National Animal Disease Center, Ames, Iowa, USA).

### 1.2. Growth of eukaryotic cells

The ST cells were cultivated in DMEM (Dulbecco's Modified Eagle Medium) supplemented with 10% fetal calf serum (FCS) (GIBCO-BRL), 50 mg/mL of gentamicin, 2 mM glutamine, and 1% nonessential amino acids.

### 1.3. Metabolic labeling with ³⁵S-methionine cysteine

To produce the virus labeled with ³⁵S-methionine cysteine, the cells were grown in monolayers in culture dishes 60 mm in diameter (Nunc). The layers were washed two times with DMEM without methionine/cysteine and we added 100 µCi of [³⁵S]-methionine/cysteine (Pro-mix-L-[³⁵S]- in vitro cell labeling mix, Amersham Pharmacia Biotech) to the culture medium. Infection with TGEV was carried out after metabolic labeling at 37°C for 15-18 h.

### 2. Viruses

### 2.1. Viruses used

The porcine transmissible gastroenteritis virus (TGEV) used belongs to the group of Purdue isolates, and was obtained in Indiana in 1946 (Doyle and Hutchings, 1946). The virus was adapted to grow in cell cultures (Haelterman and Pensaert, 1967), and was provided by E. H. Bohl (Ohio State University, Wooster Ohio). This TGEV isolate has been passaged in ST cells 115 times, and was cloned five times consecutively in the inventors' laboratory. The clone selected was labeled PUR46-CC120-MAD, abbreviated PUR46-MAD (Sánchez et al., 1990). This is an attenuated virus that grows well in cell cultures, providing titers between 10⁸ and 10⁹ PFU/mL.

In the encapsidation experiments, we used inocula of TGEV with defective particles that carried the encapsidated M39-GUS minigenome. Construction of the minigenome and production of the inocula were described earlier (Alonso et al., 2002; Izeta et al., 1999).

### 2.2. Growth and purification of TGEV

TGEV was purified by different methods. The first purification protocol was based on a method depending on its density by centrifugation through sucrose gradients. The second method was based on capture of the virus with monoclonal antibodies (MABs) specific for the membrane proteins (immunocapture or immunopurification). In the first method, the ST cells were grown in a monolayer in rotating bottles with a culture surface of 500 cm², resulting in 100 million cells per bottle. The cells were infected with inocula of TGEV with a multiplicity of infection (MOI) of 5 plague-forming units (PFU) per cell. The supernatants of the infected cells were harvested after 42 h and were clarified by centrifugation in a Sorvall GSA rotor at 6,000 rpm for 20 min at 4°C. The virus from the clarified supernatant was sedimented by ultracentrifugation through a 31% sucrose cushion in a TEN (10 mM Tris-HCl, 1 mM EDTA, 1M NaCl, pH 7.4) buffer with 0.05% Tween-20 and centrifuged through a continuous 30% to 42% sucrose gradient in TEN buffer, at 27,000 rpm in the SW60.Ti Beckmann rotor for 2 h at 4°C. The gradient was fractioned from the bottom, and the fractions containing virus were identified by ultraviolet light absorbency (260 nm). These fractions were diluted with TEN buffer. The virions were then sedimented by ultracentrifugation at 27,000 rpm for 1 h and resuspended in TNE buffer (10 mM Tris-HCl, pH 7.4, 1mM EDTA and 100 mM NaCl) to a concentration of 1 µg/µL. The purity of the virus was established by SDS-PAGE electrophoresis, visualizing the proteins of the virus by silver nitrate staining.

In the second method, the supernatants obtained from the infected cells, with or without metabolic labeling, were recovered and clarified by low-velocity centrifugation in a Microfuge (3,000 rom for 5 min). The virions were concentrated 100 times by ultracentrifugation at 27,000 rom at 4°C for 50 min in the SW60.Ti Beckmann rotor through a 30% sucrose cushion in a TNE buffer. The supernatant was removed by aspiration with a vacuum pump and the virions were resuspended in 30-40 µL of saline solution buffered with phosphate (PBS). The recovered virions were analyzed by transference and immunodetection with monoclonal antibodies or by autoradiography. Then protein A was fixed (Amersham Pharmacia Biotech) on 96 well vinyl plates (5 µg in 100 µL of PBS buffer per well) (Data Packaging Corporation) by incubation for 2 h at 37°C. The active sites of the plate were blocked with 120 µL of PBS-5% BSA buffer for 2 h at 37°C. The blocking buffer was drawn off, the wells were washed two times with 120 µL of PBS-BSA at 0.1%, and rabbit anti-rat monoclonal antibody (MAB) was added (10 µg/well). The antibodies were incubated for 1 h at 37°C. The wells were washed two times with 120 µL of PBS-0.1% BSA buffer, and we added monoclonal antibodies (MABs) specific for the N and M structural proteins of TGEV (6 µg of antibodies per well, in a final volume of 60 µL in PBS-0.1% BSA for 1 h at 37°C. The wells were washed twice with PBS-0.1% BSA buffer and the concentrated virions were added (some 5 µg in 30 µL) over a period of 12 h at 4°C. The wells were washed 10 to 15 times and the immunopurified virions were processed depending on the type of analysis (see below).

### 2.3. Purification of capsids and nucleocapsids of TGEV

The capsids of the virus were obtained from 100 or 300 µg of purified virus by dissolving the viral membranes with NP-40 nonionic detergent to a final concentration of 1 % in a buffer solution containing 100 mM Tris-HCl, 10 mM MgCl₂, pH 8 (BD buffer), in a final volume of 500 µL. The lipid envelopes were dissolved at room temperature for 15 or 30 min, under agitation. The capsids were purified by ultracentrifugation through a continuous sucrose gradient of 15% to 45% in BD buffer, or by sedimentation through a 25% sucrose cushion, in the SW60.Ti Beckmann rotor at 27,000 rpm for 50 min at 4°C. The gradient was fractionated from the bottom, and the fractions containing the capsids were.identified by SDS-PAGE electrophoresis and silver nitrate staining. The fractions with capsids were identified and visualized by electron microscope, and were diluted in a BD buffer. The capsids were sedimented by ultracentrifugation at 27,000 rpm for 50 min at 4°C in the SW60.Ti Beckmann rotor, and were resuspended in binding buffer (BU; 10 mM HEPES pH 7.4, 1 mM DTT, 10 mM MgCl₂, 50 mM NaCl, 10% glycerol, and 0.1 mM EDTA) (Homann et al., 1991) to a final protein concentration of 1 µg/µL. The purity of the capsids was analyzed by SDS-PAGE electrophoresis and silver nitrate staining.

The nucleocapsids were obtained from 100 µg of purified capsids disassembled by treatment with 400 mM KCl in a BD buffer in a final volume of 400 µL. The nucleocapsids were sedimented by ultracentrifugation at 27,000 rpm for 50 min at 4°C in the SW60.Ti Beckmann rotor, through a 22% sucrose cushion in BD buffer. The supernatant was eliminated and the nucleocapsids were resuspended in 50 µL of BU buffer to a final protein concentration of 1 µg/µL.

### 3. Bacteria

### 3.1. Bacterial strains

To clone plasmids, we used the strain *Escherichia coli* XL 1-Blue (Strategene) (Bullock et al., 1987), containing an F' episome with the gene for tetracycline resistance and a portion of the β-galactosidase gene. The genotype of this bacterial strain is: *end*A1 *hsd*R17 (rₖ₋, mₖ₋) *sup*E44 *thi*-1 *rec*A1 *gyr*A96 *rel*A1 Δ(*lac*) [F' *pro*AB *lac*I_Z_M15 *Tn*10(tetR)].

### 3.2. Preparation of competent bacteria

For amplification and production of electroporation-competent *E. coli* XL1-Blue bacteria, these bacteria were grown in a medium supplemented with tetracycline at a concentration of 20 µg/mL. We inoculated 10 mL of SB medium (30 g/L tryptone, 20 g/L yeast extract, 10 g/L MOPS) with 20 mM glucose with a colony from a fresh plate, and the bacteria were incubated for 12 h at 37°C and under agitation. With 2 mL of this culture, we inoculated 1 L of SB medium supplemented with 2M glucose, and the bacteria were grown at 37°C to an optical density of 600 nm, between 0.8 and 0.9 absorbance units. The culture was cooled on ice for 20 min, and the bacteria were centrifuged in the Sorvall GSA rotor at 4,000 G for 15 min at 4°C. The bacteria were resuspended cold in 1 L of 10% glycerol. The bacteria were centrifuged again and resuspended in 500 mL of 10% glycerol. The bacteria were sedimented and resuspended in 250 mL of 10% glycerol. Finally, the bacteria were centrifuged and resuspended in 3 mL of 10% glycerol. The bacteria were divided into aliquote parts of 100 µL and were kept at -70°C until they were used for electroporation. The transformation efficiency of the bacteria was calculated by electroporation of a pBluescript plasmid with a known concentration as a reference, and was reproducibly at about 10⁹ colonies/µg of DNA.

### 3.3. Transformation of bacteria by plasmid electroporation

We used 50 µL of transformation-competent bacteria. We added 1 µL of each reaction mixture, or 10 ng of purified plasmid to the bacteria and incubated on ice for 1 min. Then, the bacteria with plasmids were transferred to 0.2 cm electroporation trays (Bio-Rad), and were transformed by a 2.5 kV electric pulse, 25 µF and 200 Ω in a "Gene Pulser" electroporator (Bio-Rad). We then added 1 mL of cold LB medium, and the bacteria were incubated at 37°C under agitation for 1 h. Between 50 and 100 µL of the suspension of transformed bacteria were seeded in Petri dishes with LB (Luria-Bertani medium) in a solid medium (15 g/L agar) supplemented with ampicillin (100 µg/mL). The bacteria grew for 16 h at 37°C.

For production and purification of plasmids, the transformed bacteria with plasmids which conferred ampicillin resistance were frown from an isolated colony on a plate, in a liquid LB medium supplemented with 100 µg/mL of ampicillin.

### 4. Plasmids

### 4.1. Plasmids from cloning of PCR products

The pGEMT (Promega) plasmid was used to clone PCR products. This plasmid contains the T7 and SP6 bacteriophage promoters separated by the LacZ gene, interrupted by two protruberant T sequences between which was situated a multicloning sequence. This plasmid confers ampicillin resistance for its selection.

### 4.2. Expression plasmids in eukaryotic cells

For cloning and expression of genes in eukaryotic cells, we used the plasmid pcDNA3 (Invitrogen). This plasmid contains the cytomegalovirus (CMV) immediate early genes and T7 promoters followed by a multicloning sequence and the polyadenylation sequence from the bovine growth hormone (BGH). This plasmid confers resistance to the antibiotics ampicillin and geniticin for its selection in both bacterial and eukaryotic cells.

The plasmid pSL1190 (Pharmacia) was used for cloning and expression of minigenomes derived from TGEV under control of the CMV promoter (Alonso et al., 2002; Izeta et al., 1999).

### 5. Manipulation of DNA

### 5.1. Cloning and restriction enzymes

For manipulation and cloning of DNA, the restriction enzymes BamHI, NdeI, SalI, ApaI and ClaI were acquired from Boehringer Mannheim or from New England Biolabs. For dephosphorylation of the DNA terminals, we used shrimp alkaline phosphatase (SAP) (USB). For DNA ligation, we used T4 phage DNA ligase (New England Biolabs). All the treatment with restriction enzymes, dephosphorylation, and DNA ligation were carried out by protocols that have been described previously (Sambrook et al., 1989).

### 5.2. Polymerase chain reaction (PCR)

To amplify DNA from a matrix, frequently plasmids, we used between 50 and 100 ng of plasmid and added the corresponding oligonucleotides (10 µM), 0.25 mM deoxynucleotides triphosphate (ATP, GTP, TTP, and CTP), 1.25 mM MgCl₂, PCR buffer (10 mM Tris-HCl, pH 8.3, 50 mM KCI) and 2.5 U of Taq DNA polymerase (*Thermus aquaticus*) (Perkin-Elmer), in a final volume of 50 µL. The reactions were carried out in the GeneAmp PCR System 9600 thermocycler from Perkin Elmer.

### 5.3. Separation of DNA by agarose gel electrophoresis

To separate DNA fragments, we used D-1 Media EEO (Pronadisa) 1% agarose gels with ethyl bromide (1 µg/mL) in a 1X TAE buffer (40 mM Tris-acetate, 1mM EDTA).

### 5.4. Purification of DNA

The plasmids grown in bacteria in the presence of the selection antibiotics were purified using the "CONCERT™ Rapid Plasmid Miniprep System" (Life Technologies) for preparation of small quantities of plasmid DNA, and the system "Qiafilter Midi-Plasmic Kit" (Qiagen) for preparations of intermediate quantities of plasmid DNA. The DNA obtained from agarose gels was purified using the system "QiaEx II Gel Extraction Kit" (Qiagen). Purification of the PCR products was carried out by means of the system "QIA quick PCR Purification Kit" (Qiagen). In all cases, we followed manufacturers' instructions.

### 6. RNA analysis

### 6.1. Extraction of cellular RNA

For analysis of the RNA produced in infections with TGEV clone PUR46-MAD, we infected confluent monolayers of ST cells grown in 60 mm diameter culture plates (NUNC) with viral inocula at a MOI of 1. The cells were lysed at 12 hpi [hours post infection] with 1 mL of ULTRASPECT reagent, following the protocol provided by the commercial firm (Biotecx) based on the method of acid phenolization in the presence of guanidine isothiocyanate. The RNA was purified and resuspended in 40 µL of water treated with DEPC [diethyl polycarbonate] and 20 U of RNAse inhibitor (Roche).

### 6.2. Extraction of RNA from virions

We used 10 µg of virus for analysis of the RNA encapsidated in virions that were partly and totally purified by centrifugation through a 31 % sucrose cushion in a continuous sucrose gradient, respectively. The RNA was extracted with 100 µL of the reagent ULTRASPECT in the presence of 40 µg of yeast cell RNA. The RNA was purified according to manufacturer's instructions, and was resuspended in 40 µL of DEPC water with 20 U of RNAse inhibitor.

For analysis of the RNA encapsidated in virions that were purified by immunocapture, once the virions were immunocaptured and washed, we added 10 µg of yeast RNA per well to favor precipitation of viral RNA, and extracted the RNA from the virions with 100 µL of ULTRASPECT reagent per well. The RNA was purified and resuspended as described above.

### 6.3. Analysis by RT-PCR

For detection of genomic RNA and each of the subgenomic RNAs of the virus [sic; probably, by] obtaining complementary DNA, we performed reverse transcriptase (RT) reactions with specific antisense (-)oligonucleotides that hybridized with each of the ORFs of each subgenomic RNA (Table 1). The reactions occurred in a volume of 20 µL at 42°C for one hour in the presence of 0.25 mM deoxynucleotide triphosphates (ATP, GTP, TTP, CTP), 1 mM DTT, 2.5 mM MgCl₂, PCR buffer and 6 U of reverse transcriptase from the avian myeloblastosis virus (AMV) (Seikagaku).

For detection of the M39-GUS minigenome and the subgenomic RNA derived from the M39-GUS minigenome, we used the GUS oligonucleotide (5'-GACCCACACTTTGCCGTAATGAGTGACCGCA-3') that hybridizes specifically with the gus gene present in the minigenome and with its messenger RNA, or the GUS 297 oligonucleotide (5'-GACCCACACTTTGCCGTAATGAG-3').

Amplification of the DNA was accomplished by PCR using as the matrix 10 µL of the RT reactions. For amplification of the genomic RNA, we used oligonucleotide 1.7, which hybridizes in positions 1,660 and 1,676 of the genomic RNA, or oligonucleotide 1.8, which hybridizes in positions 1,849 and 1,870. For amplification of the subgenomic RNAs, we used each of the antisense oligonucleotides described above together with an oligonucleotide having the leader sequence of TGEV (Leader oligonucleotide, 5'-AGATTTTGTCTTCGGACACCAACTCG-3'). The M39-GUS minigenome was detected using the antisense GUS or GUS 297 nucleotide together with oligonucleotide 19949, which hybridizes between positions 3,228 and 3,245 in the minigenome.

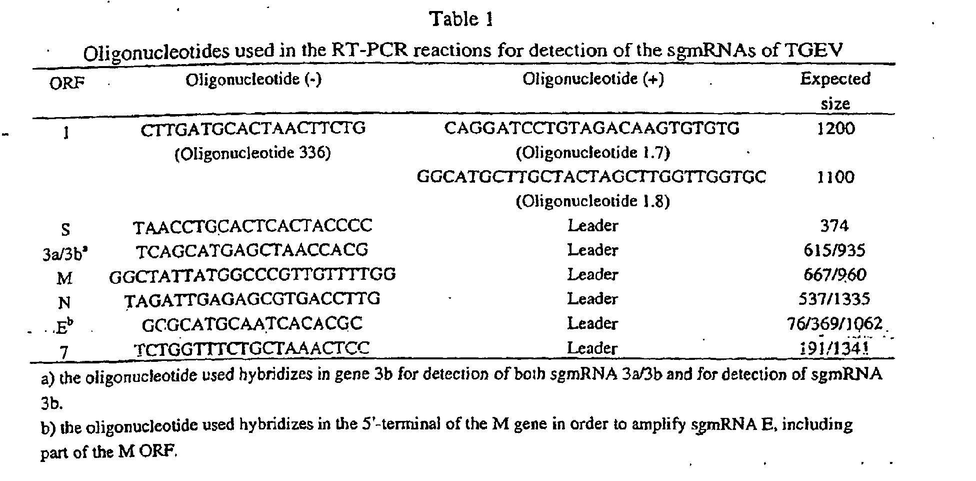

The PCR reactions occurred during 25-35 cycles at a Tm of 50°C and 50 sec of elongation at 72°C. The PCR products were analyzed by agarose gel electrophoresis.

### 7. Analysis of proteins

### 7.1. Electrophoresis of proteins in polyacrylamide gels (SDS-PAGE)

All the protein samples were analyzed in gels of 10% polyacrylamide in the presence of sodium dodecyl sulfate (SDS-PAGE electrophoresis) or in gels in a linear polyacrylamide gradient (5% to 15%) (Laemmli, 1970). The proteins were stained in the gel with the reagent "GelCode Blue Stain" (PIERCE) or with a silver nitrate (Ansorge, 1985).

### 7.2. Transfer of proteins to nitrocellulose membranes and immunodetection with specific antibodies (transfer and immunodetection, or Western type transfer

For detection of the proteins by transference and immunodetection, the proteins separated by SDS-PAGE electrophoresis were transferred to nitrocellulose membranes with a Mini Protean II electrotransfer apparatus (Bio-Rad) at 150 mA for 2 h in a transfer buffer (25 mM Tris-192 mM glycine, 20% methanol, pH 8.3). The membranes were blocked for 2 h with 5% powdered skim milk (Nestlé) in TBS buffer (20 mM Tris-HCl, pH 7.5,150 mM NaCl) at room temperature. The membranes were incubated with the specific MABs for the S, N, M or β-glucuronidase (GUS) proteins. The bound antibody was detected with rabbit antibodies specific for rat IgG immunoglobulins or with goat anti-rabbit immunoglobulins, conjugated with radish peroxidase, using the ECL chemoluminescence system (Amersham Pharmacia Biotech).

### 7.3. Antibodies

The specificity of the MABs 5B.H1, 9D.B4, 3D.E3, 3B.B3, 3D.C10, 25.22 and 1A6 has been characterized previously (Charley and Laude, 1988; Jiménez et al., 1986; Laude et al., 1992; Martín-Alonso et al., 1992; Risco et al., 1995; Suñe et al., 1990; Wesley et al., 1988; Woods et al., 1987). The MABs 9D.B4, 3B.B3 and 3D.E3 specifically recognize the carboxy terminal of the M protein of TGEV, and MABs 25.22 and 1A6 are specific for the amino terminal (Charley and Laude, 1988; Laude et al., 1992; Wesley et al., 1988; Woods et al., 1987). The MABs 3D.C10 and 5BH1 recognize the N and S proteins of TGEV, respectively (Jiménez et al., 1986; Martin-Alonso et al., 1992). The rabbit antiserum specific for the GUS protein was acquired from 5 Prime → 3 Prime, Inc. The F fraction (ab)₂ of a rhodamine-conjugated goat anti-rat immunoglobulin antibody was acquired from Cappel. The rabbit anti-rat immunoglobulin antibody used in immunopurification of TGEV was acquired from Cappel.

### 8. Estimation of the molar ratio between M and N proteins in purified virions and capsids

Purified viruses and capsids (2 µg) were analyzed by SDS-PAGE electrophoresis in polyacrylamide gradients (5% to 20%) and the structural proteins were stained with silver nitrate. The protein bands were quantified by densitometry using the program "Gel Documentation System 2000" (Biorad). We used seven independent virus purifications to estimate the molar ratio between the M and N proteins in both virions and capsids. The normal distribution of the M/N molar ratio was demonstrated with the chi-square test (Martin Andrés and Luna del Castillo, 1994). The M/N stoichiometric ratio between virions and the capsids was compared by Wilcoxon's non-parametric t-test (Motulsky, 1995).

### 9. Direct binding of MABs to the surface of the TGEV virion

To study the domains of the M protein exposed on the surface of TGEV, we incubated 50 µg of purified virus with 5 µg of MABs 25.22, 9D.B4, and 3D.E3 specific for the M protein, and MAB 3D.C10, specific for the N protein, in a final volume of 500 µL of TNE buffer with 0.05% Tween-20 for a period of 2 h at 4°C. The virions were sedimented by ultracentrifugation through a 30% sucrose cushion in TNE buffer with 0.05% Tween-20, at 27,000 rpm in the SW60.Ti Beckmann rotor for 1 h at 4°C. The virions were resuspended in 50 µL of TNE buffer. The TGEV was incubated with the MAB 3D.C10 in the absence and the presence of the detergent 1%NP-40 as a control of the structural integrity of the virus. The viruses with the MABs bound to their surface were analyzed by SDS-PAGE electrophoresis, and the immunoglobulin chains of the MABs were detected directly by transfer and immunodetection with a peroxidase-conjugated rabbit anti-rat immunoglobulin antibody.

To verify whether the M protein present in the preparations of the purified capsids came from the viral envelope, we added NP-40 to a final concentration of 1% to virus preparations with MABs specific for the M protein bound to the surface, and the mixture was agitated for 10 min to break the viral membranes. The released capsids were sedimented by ultracentrifugation through a 25% sucrose cushion in TNE buffer with 1 % NP-40, at 27,000 rpm for a period of 50 min at 4°C in the SW60. Ti Beckmann rotor. The capsids were recovered in 30 µL of loading solution for SDS-PAGE electrophoresis. The presence of the immunoglobulin chains was studied in the virions and in the capsids by transference and immunodetection.

### Example 1

### Identification of the encapsidation signal in the genome of TGEV

### 1. Materials and methods

### 1.1. Experimental design

The experimental design for identification of the ES in TGEV was based on detection of the encapsidation of RNAs by means of immunocapture of virions coupled to detection of the RNAs by RT-PCR. We studied the RNA sequence that would have to be added to a subgenomic messenger RNA for it to encapsidate in virions. To do this, we took as the basis the M39-GUS minigenome, which encapsidates specifically, in contrast with the subgenomic messenger RNA for the gus gene (sgmGUS or RNA-GUS). The sequences that might contain the ES (Figure 1) were cloned after the conserved sequence CTAAAC (CUAAAC in the RNA) of the transcription regulatory sequence (TRS) of the minigenome (Figure 2). The messenger RNAs derived from the minigenome would be formed by the leader sequence fused with the conserved sequence (CUAAAC) followed by the rest of the minigenome sequence. These.minigenomes would produce subgenomic mRNAs that could potentially contain the region responsible for encapsidation of the genome, and these messengers, in theory, would be encapsidated in virions.

### 1.2. Cloning the sequences potentially containing the ES in the M39-GUS minigenome

To select the sequences that might potentially contain the ES, we started from the M33 minigenome, the smallest that can be efficiently recovered in cell cultures (Figures 1 and 3). The ES was restricted to two discontinuous zones in the genome of the virus, present in the 5'-region of the M33 minigenome. The first zone, region A, is made up of the first 2,144 nucleotides of the 5'-terminal of the virus, and the second, region B, proceeds from 568 nt of the overlapping sequence between ORFs 1a and 1b, between nucleotides 12,195 and 12,763 of the TGEV genome. Region A, in turn, was subdivided into fragments of 550 nt, which overlapped 50 nt (Figure 1). These fragments were amplified by PCR using synthetic oligonucleotides (Table 2) which flanked each of the regions, introducing SalI restriction sequences in the terminals of the amplified fragments. The PCR fragments were digested directly with the SalI enzyme, and were cloned in the plasmid pSL1190 (pCMV)-M39-GUS cut with the SalI enzyme (Figure 2). The plasmids obtained were analyzed by sequencing, and those containing regions A1, A2, A3, A4, and B in phase were selected (Figure 2).

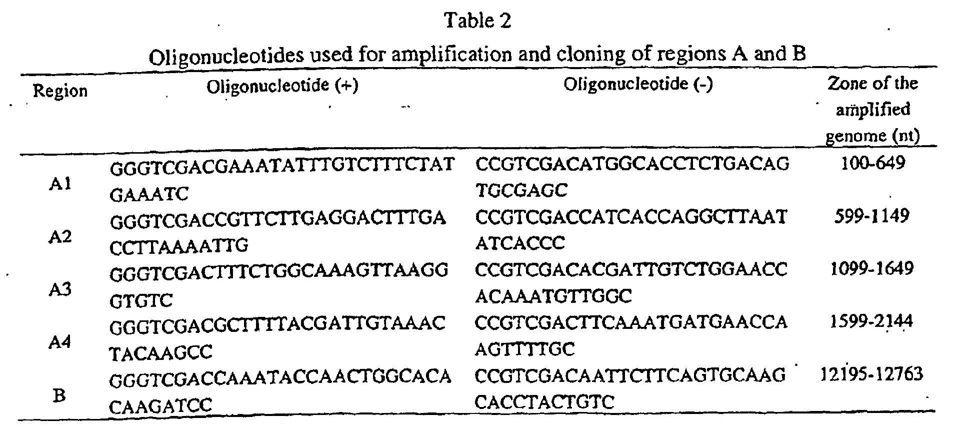

### 1.3. Recovery of the minigenomes generated

A quantity of 5 µg of each plasmid including each one of the minigenomes under control of the CMV promoter was transfected into swine testicle (ST) epithelial cells. The cells were infected with TGEV at a multiplicity of infection (MOI) of 5 at 12 h post-transfection. The supernatants obtained from the transfection/infection were used to infect confluent ST cells (passage 1) and the supernatant was passed into ST cells two more times (passages 2 and 3) (Figure 4).

### 1.3.1. Detection of minigenomes and subgenomic mRNAs in ST cells and in virions

The RNA from the infected cells was purified in passage 3, and the minigenomes and subgenomic mRNAs of the minigenomes were detected by RT-PCR. To detect the minigenome and the subgenomic mRNAs, we used an RT reaction using the GUS 297 oligonucleotide (5'-GACCACACTTTGCCGTAATGAG-3'). The minigenome was amplified by PCR using the GUS 297 and 19949 oligonucleotides (5'-CTTGGTGGATCTGTTGCC-3'). The subgenomic mRNA was amplified by PCR using the GUS 297 and leader oligonucleotides. The products of the RT-PCRs were analyzed by agarose gel electrophoresis together with markers for molecule size.

The virions generated from the infected cells (passage 3) were concentrated and immunopurified. The analysis of the minigenomes and mRNAs was made by RT-PCR.

### 2. Results

### 2.1. Detection of genomic and subgenomic RNAs in ST cells infected with TGEV

First we perfected the detection of subgenomic RNAs of the virus in cells infected with TGEV. We designed amplifications by RT-PCR specific for each of the subgenomic RNAs, using the specific oligonucleotides described above. The products of the RT-PCR reactions were analyzed by agarose gel electrophoresis. The major products of the RT-PCR reactions shows the expected size for each of the viral RNAs. These products were sequenced to confirm that they corresponded to the various mRNAs of the TGEV (Figure 5).

### 2.2. Detection of the genomic and subgenomic RNAs in preparations of purified viruses

### 2.2.1 Purification of the TGEV virions

To verify whether detection of subgenomic mRNAs in purified virions depended on the purification process, i.e., to determine whether they encapsidated specifically or whether contaminations were present, we obtained preparations of purified viruses using three different methods. The first method consisted of concentrating the virus by ultracentrifugation through a 31% sucrose cushion, starting with supernatants of infected ST cells. In the second method, in addition to the concentration through the sucrose cushion, the virus was purified by centrifugation in a continuous sucrose gradient. In the third method, we changed strategy. The virus that had been previously concentrated by ultracentrifugation was purified by' immunocapture with MAB 25.22, specific for the amino terminal of the M protein. The purity of the viral preparation was estimated by SDS-PAGE electrophoresis analysis and silver nitrate staining in the case of the first two methods, and by SDS-PAGE electrophoresis and fluorography in the third method, using radioactively labeled virus (Figure 6). In the concentrated virus, we detected the three major structural proteins of the virus and high-molecular weight contaminating proteins. In the virus preparations purified by sucrose gradient and by immunocapture, no contaminating proteins were detected.

### 2.2.2. Detection of genomic and subgenomic RNAs in purified virus preparations

We then investigated the presence of genomic and subgenomic RNAs in the preparations of purified viruses that were amplified by RT-PCR (Figure 7). In the concentrated virus, we detected the genomic and subgenomic RNAs S, E, and M. In the virus purified by sucrose gradient, we detected the genome and mRNAs E and M, but not S, previously detected. In the virus purified by immunocapture, we detected the genome and mRNA E. When the number of washings was increased from 10 to 15 times without detergent during immunocapture, or including the detergent Tween-20 at two concentrations (0.05% and 0.5%), only the genomic RNA was detected. Because the detection of the mRNAs of the virus depended on the virus purification process, and since we created extremely pure virions, in which the presence of mRNAs could not be identified, it was concluded that the mRNAs detected in purified virus were contaminating and had not been encapsidated.

### 2.3. Detection of the M39-GUS minigenome and the subgenomic sgmGUS in infected cells and in virions purified by immunocapture

To determine the encapsidation of RNAs of minigenomes that were not lost in successive passages in cell cultures (Alonso et al., 2002), we used a system based on infection of ST cells with preparations of TGEV containing virions with the M39-GUS minigenome. This minigenome presents the necessary signals for its replication and encapsidation, and it incorporates a transcriptional unit composed of a TRS derived from the N gene followed by the gus tracer gene. This mini genome replicates in the infected cells, and also transcribes the subgenomic mRNA for the gus gene (sgmGUS or mRNA-GUS). We designed a specific detection strategy for the M39-GUS minigenome and its messenger by RT-PCR (Figure 8A). For specific detection of mRNA we used an oligonucleotide that hybridized with the gene gus together with an oligonucleotide complementary to the leader sequence of the viral genome and of the subgenomic mRNAs. In theory, by using this RT-PCR, we should be able to detect the M39-GUS minigenome, although under the conditions in which RT-PCR was carried out (i.e., short elongation times) only the GUS messenger was amplified, with 374 bp, but not the product derived from the minigenome with a theoretical length of 3,700 bp.

The presence of the genome, minigenome, and the mRNA-GUS was investigated in infected cells and in virions purified by immunocapture from the supernatants of these infected cells (Figure 8B). In the cells, we detected the RNAs corresponding to the genome, to the M39-GUS minigenome, and to the mRNA-GUS. The nature of these bands was confirmed by sequencing the products of the RT-PCR reactions. In the reaction for detection of the mRNA-GUS, an unexpected product appeared with an approximate size of 1,070 nt. This product was sequenced and it was established that it was generated in the RT-PCR reaction by nonspecific hybridization of the GUS oligonucleotide with the 5'-terminal of the genome and the minigenome. In the virions purified by immunocapture, we detected the genomic RNA, the M39-GUS minigenome, but not the GUS messenger, in contrast to the results obtained in the infected cells. The nonspecific band of 1,070 nt was also detected in the immunopurified virions. In subsequent analyses, we used the GUS 297 oligonucleotide, which did not hybridize nonspecifically with the genome.

### 2.4. Identification of the ES in the genome of TGEV

Once it has been demonstrated that the subgenomic mRNAs do not encapsidate in virions, in contrast to the minigenomes, we designed constructs based on the M39-GUS minigenome that produced subgenomic mRNAs including sequences that potentially contained the ES. The smallest mini genome derived from TGEV that was recovered efficiently in cell cultures was M33 (Figure 9A), which suggested that this minigenome included the ES in the 5'-terminal of the minigenome. This ES, in theory, came from the discontinuous zones of the viral genome, A and B, which were located in the first 2,144 nt of the viral genome or in the 568 nt of the overlapping region between genes 1a and 1b (from nucleotide 12,195 to nucleotide 12,763), respectively (Figure 9B).

To locate the position of the ES, zone A was subdivided into four fragments of 550 nt, which overlapped for a distance of 50 nt (Figure 9B). These fragments, in addition to region B, were amplified by PCR, and each fragment was cloned in the M39-GUS minigenome directly under the control of the optimized TRS, following the conserved sequence 5'-CUAAAC-3' and the sequence that flanks the 3'-side of the CS [sic; GUS] sequence (5'-ACGTCGACGA-3') (Figure 10).

ST cells were transfected with the minigenomes generated, where they were transcribed under the CMV promoter. The minigenomes generated were recovered by infection with the complementary TGEV. The virions generated in the transfected and infected ST cells were passaged three times in cell cultures to amplify the minigenomes.

The presence of the genome, minigenomes, and mRNAs was studied in the infected cells and in the purified virions. Independent of the sequence introduced in the minigenomes, the genomes, the minigenomes, and their messengers were detected in the infected cells (Figure 11). However, in the virions purified from the infected cells, only the mRNA was detected when the virions were produced in cells that were transfected with the M39-GUS-A1 minigenome. In contrast, in the rest of the viruses purified from the other transformed cells, only the genome and the minigenome were detected, but not the mRNAs.

In the case of the virions originating from the infected cells in the presence of the minigenome containing the A1 sequence, the most abundant RNA was mRNA-A1, i.e., the one that included the genome sequence from nucleotide 100 to 649. Even when comparing the products of the RT-PCR reactions with the rest of the constructs, we repeatedly observed that in the case of construct A 1 the viral genome was present in a smaller quantity than in the rest of the constructs, as determined by RT-PCR. These results strongly suggested that mRNA-A1 was encapsidated very efficiently, inhibiting encapsidation of the viral genome itself.

The results obtained indicate that the ES of the virus was located in the 5'-terminal of the genome, between nucleotides 100 and 649, since their insertion in a subgenomic mRNA that does not encapsidate resulted in its efficient encapsidation into virions. This result is different from the result obtained in the coronaviruses MHV and BCoV, in which the ES is distant from. the 5'-terminal of the virus, at about position 20,300 of the genome of these viruses (Figure 12).

### 3. Discussion

The TGEV encapsidated the genomic RNA and the minigenomes, but not the viral mRNAs. To identify the ES, we used a strategy of positive action - a strategy in which the encapsidation of the mRNA was shown as a consequence of the insertion of encapsidation signals into them, This strategy consisted of insertion of regions originating from the 5'-terminal of the M33 minigenome, under the control of the TRS sequence of the M39-GUS minigenome. In this way, in the cells that were infected in the presence of these minigenomes, mRNAs were produced with the indicated sequences cloned in the minigenome. These constructs were recovered in ST cells transfected and infected with complementary TGEV. In the virions released from the infected cells, we detected only the messengers containing the sequence included between positions 100 to 649 of the genome, indicating that the ES was found in this RNA fragment. Since these encapsidation signals are smaller than 100 nt, as described in the MHV coronavirus (Fosmire et al., 1992), the minimum sequences necessary for encapsidation of RNAs in TGEV remain to be determined.

The genomic RNA was the most abundant in purified viruses originating from cells infected with viruses in the presence of minigenomes. However, when the minigenomes contained the capacity to transcribe mRNAs which included the ES, the most abundant RNA in purified viruses was mRNA. This result indicated that the messenger RNA that contained the ES was incorporated into the virions very effectively, at the expense of encapsidation of the genomic RNA and the minigenome that generated it. Possibly, the smaller quantity of genomic RNA in the virions purified in the presence of the M39-GUS-A1 minigenome and its mRNA was due to the competition for encapsidation among the viral genome, the minigenome, and the mRNA.

Encapsidation might be, in principle, associated with replication of the genome, as is the case with poliovirus and flavivirus (Khromykh et al., 2001; Nugent et al., 1999). However, It is unlikely that this is the case with the coronaviruses, because ES-mediated encapsidation of mRNAs in the case of TGEV and in the coronavirus MHV as well (Woo et al., 1997) is independent of the RNA replication, since the mRNAs do not replicate themselves (the mechanism for replication and transcription has different requirements in coronaviruses) (Bos et al., 1997; Woo et al., 1997). However, we cannot exclude the possibility that encapsidation of mRNAs in those have had artificial introduction of ES is associated with transcription of these mRNAs, which is the mechanism by which they are synthesized (Baker and Lai, 1990; Sawicki and Sawicki, 1998; van Marle et al., 1999).

The results obtained indicated that the ES of TGEV is located between nucleotides 100 and 649 of the viral genome. This is surprising, since the ES in the coronavirus MHV was situated at 1.4 kb from the 3'-terminal of the replicase gene (Fosmire et al., 1992), i.e., a zone that is almost 20,000 nt from the one described here for the ES in the TGEV genome. The reason for this difference in location of the ES is unknown.

The encapsidation signals in different viruses present a defined secondary structure, with hairpin RNA recognized specifically by the viral nucleoproteins (Fosmire et al., 1992; Kim et al., 1998; Woo et al., 1997; Zhong et al., 1992).

The localization of the ES in the TGEV genome is of great practical and scientific importance. As an illustration, viral vectors can be developed which are useful for development of vaccines and gene therapy derived from TGEV in which, as a safety measure to prevent propagation of the vector in vivo, genes essential to the assembly of the virus have been deleted (Figure 13). To increase the level of biosafety of the vector, once the ES is surveyed, it can be relocated from the 5'-terminal of the genome to the 3'-terminal of the genome, immediately to the side of the deleted gene (Figure 13). This will prevent regeneration of the infectious virus from a recombination between the vector and a field virus. If recombination should occur, the field virus will acquire the 3'-terminal of the vector, recovering the deleted genes. However, this process would involve the loss of the ES, and it would not be able to propagate. Only in the case of a double recombination would the vector recover both the ES and the deleted genes. However, a triple recombination that would involve recovery of two deleted essential genes, in addition to the ES, is a low frequency event (recombination frequency about 1 x 10⁻⁴ x 1 x 10⁻⁴ x 1 x 10⁻⁴ = 1 x 10⁻¹²) (Sánchez et al., 1999), which would add an additional safety component to the vector presenting deletions of essential genes exclusively (Figure 13).

The identification of the ES of the genome allows the study of the encapsidation mechanisms of coronaviruses and the generation of new biosafe viral vectors for the design of new vaccines and for gene therapy applicable to animal and human health.

### Bibliography

Alonso, S., Izeta, A., Sola, I. and Enjuanes, L. (2002). Transcription regulatory sequences and mRNA expression levels in the coronavirus transmissible gastroenteritis virus. J. Virol. 76:1293-1308.
Ansorge, W. (1985). Fast and sensitive detection of protein and DNA bands by treatment with potassium permanganate. J. Biochem. Biophys. Methods 11:13-20.
Baker, S. C. and Lai, M. M. C. (1990). An in vitro system for the leader-primed transcription of coronavirus messenger RNAs. EMBO J. 9:4173-4179.
Bos, E. C. W., Dobbe, J. C., Luytjes, W. and Spaan, W. J. M. (1997). A subgenomic mRNA transcript of the coronavirus mouse hepatitis virus strain A59 defective interfering (DI) RNA is packaged when it contains the DI packaging signal. J. Virol. 71:5684-5687.
Bullock, W., Fernández, J.M. and Short, J.M. (1987). XL1-Blue: a high efficiency plasmid transforming recA *E.coli* strain with P-galactosidadse selection. Biotechniques 8:26-27.
Charley, B. and Laude, H. (1988). Induction of alpha-interferon by transmissible gastroenteritis coronavirus: Role of transmembrane glycoprotein El. J. Virol. 62:8-10.
Cologna, R and Hogue, B. G. (2000). Identification of a bovine coronavirus packaging signal. J. Virol. 74:580-583.
Doyle, L.P. and Hutchings, L.M. (1946). A transmissible gastroenteritis in pigs. J. Amer. Vet. Med. Assoc. 108:257-259.
Fosrnire, J. A., Hwang, K. and Makino, S. (1992). Identification and characterization of a coronavirus packaging signal. J. Virol. 66:3522-3530.
Haelterman, E.O. and Pensaert, M.B. (1967). Pathogenesis of transmissible gastroenteritis of swine. Proc. 18th World Vet. Congreso 2:569-572.
Homann, H.E., Willenbrink, W., Buchholz, CJ and Neubert, W.J. (1991). Sendai virus protein-protein interactions studied by a protein-blotting protein-overlay technique:mapping of domains on NP protein required for binding to P protein. J. Virol. 65:1304-1309.
Izeta, A., Smerdou C., Alonso, S., Penzes, Z., Méndez, A., Plana-Durán, J. and Enjuanes, L. (1999). Replication and packaging of transmissible gastroenteritis coronavirus-derived synthetic minigenomes. J. Virol. 73:1535-1545.
Jiménez, G., Correa, I., Melgosa, M.P., Builido, M.J. and Enjuanes, L. (1986). Critical epitopes in transmissible gastroenteritis virus neutralization. J. Virol. 60:131-139.
Khromykh, A. A., Vamavski, A. N., Sedlak, P. and Westaway, E. G. (2001). Coupling between replication and packaging of flavivirus RNA: Evidence derived from the use of DNA-based full-length cDNA clones of Kunjin Virus. J. Virol. 75:4633-4640.
Laemmli, U.K. (1970). Cleaqvage of structural proteins during the assembly of the head of bacteriophage T4. Nature 227:680-685.
Laude, H., Gelfi, J., Lavenant, L. and Charley, B. (1992). Single amino acid changes in the viral glycoprotein M affect induction of alpha interferon by the coronavirus transmissible gastroenteritis virus. J. Virol. 66:743-749.
Martin-Alonso, J.M., Balbin, M., Garwes, D.J., Enjuanes, L., Gascón, S. and Parra, F. (1992). Antigenic structure of transmissible gastroenteritis virus nucleoprotein. Virology 188:168-174.
Martín Nndrés [sic; Andrés], A. and Luna del Castillo, J. d. D. (1994) Biostatistics for health sciences. 4^{th} Edition. Norma Publishing Company, Inc. Madrid
McClurkin, A.W. and Noman, J.O. (1996). Studies on transmissible gastroenteritis of swine. II. Selected characteristics of a cytopathogenic virus common to five isolates from transmissible gastroenteritis. Can. J. Comp. Med. Vet. Sci. 30:190-198.
Motulsky, H. (1995). Intuitive Biostatistics. Oxford University Press. New York.
Nugent, C. I., Johnson, K. L., Sarnow, P. and Kirkegaard, K. (1999). Functional coupling between replication and packaging of poliovirus replicon RNA. J. Virol. 73:427-435.
Risco, C., Antón, I. M., Suííé, C., Pedregosa, A. M., Martí-Alonso, J. M., Parra, F., Carrascosa, J. L. and Enjuanes, L. (1995). Membrane protein molecules of transmissible gastroenteritis coronavirus also expose the carboxy-terminal region on the external surface of the virion. J. Virol. 69:5269-5277.
Sambrook et al. (1989). Molecular Cloning: A Laboratory Manual. Ed Cold Spring Harbor Laboratory.
Sánchez, C. M., Jiménez G., Laviada, M.D., Correa, I., Suñé, C., Bullido, M.J., Gebauer, F., Smerdou, C., Callebant, P., Escribano, J.M. and Enjuanes, L. (1990). Antigenic homology among coronaviruses related to-transmissible gastroenteritis virus. Virology 174:410-417.
Sánchez, C. M., Izeta, A., Sánchez-Morgado, J. M., Alonso, S., Sola, I., Balasch, M., Plana-Durán, J. and Enjuanes, L. (1999). Targeted recombination demonstrates that the spike gene of transmissible gastroenteritis coronavirus is a determinant of its enteric tropism and virulence. J. Virol. 73:7607-7618.
Sawicki, S. G. and Sawicki, D. L. (1986). Coronavirus minus-strand RNA synthesis and effect of cycloheximide on coronavirus RNA synthesis. J. Virol. 57:328-334.
Suñe, C., Jiménez G., Correa, I., Bullido, M.J., Gebauer, F., Smerdou, C. and Enjuanes, L. (1990). Mechanisms of transmissible gastroenteritis coronavirus neutralization. Virology 177:559-569.
van Marle, G., Dobbe, J. C., Gultyaev, A. P., Luytjes, W., Spaan, W. J. M. and Snijder, E. J. (1999). Arterivirus discontinuous mRNA transcription is guided by base pairing between sense and antisense transcription-regulating sequences. Proc. Nat. Acad. Sc. USA 96:12056-12061.
Wesley, RD., Woods, RD., Correa, I. and Enjuanes, L. (1988). Lack of protection *in vivo* with neutralizing antibodies to transmissible gastroenteritis virus. Vet. Microbiol. 18:197-208.
Woo, K, Joo, M., Narayanan, K., Kim, K. H. and Makino, S. (1997). Murine coronavirus packaging signal confers packaging to nonviral RNA. J. Virol. 71:824-827.
Woods, RD., Wesley, RD. and Kapke, P.A. (1987). Complement-dependent neutralization of transmissible gastroenteritis virus by monoclonal antibodies. Adv. Exp. Med. Biol. 218:493-500.
Zhong, W. D., Dasgupta, R and Rueckert, R. (1992). Evidence that the packaging signal for nodaviral RNA2 Is a bulged stem-loop. Proc. Natl. Acad. Sci. USA 89:11146-11150.
Zucker, M. (1 989a). Computer prediction of RNA structure. Methods Enzymol. 180:262-288.
Zucker, M. (1989b). On finding all suboptimal foldings of an RNA molecule. Science 244:48-52.

## Claims

1. A nucleic acid sequence selected from:
a) a sequence of nucleotides consisting of from nucleotide 100 to nucleotide 649 of the genome of a group 1 coronavirus, said nucleotide sequence including the encapsidation sequence of said coronavirus;
b) a nucleic acid sequence analogous to the sequence defined in a) that contains the encapsidation sequence of a group 1 coronavirus;
c) a nucleic acid sequence complementary to either of said sequences a) or b), and
d) a nucleic acid sequence with a secondary structure similar to that of an encapsidation sequence (ES) of a group 1 coronavirus, independent of the primary nucleotide sequence.

2. Sequence according to Claim 1, wherein said nucleic acid is any molecule of DNA or RNA.

3. Sequence according to Claim 1, wherein said group 1 coronavirus is selected from the group made up of porcine, canine, feline, and human coronaviruses.

4. Sequence according to Claim 1, made up of the nucleotide sequence shown in SEQ. ID. No.: 1, or by a fragment thereof, which contains the encapsidation sequence of the porcine transmissible gastroenteritis virus (TGEV).

5. A nucleic acid construct that includes a nucleic acid sequence according to any of Claims 1-4, together with, optionally, a transcription promoter sequence.

6. A plasmid that contains said nucleic acid sequence according to any of Claims 1-4, or said nucleic acid construct according to Claim 5.

7. A vector that includes
a nucleic acid sequence (I) selected from (i) a nucleic acid sequence corresponding to the complete genome, or a part thereof, of a group 1 coronavirus, and (ii) a nucleic acid sequence of another virus that has a sequence identity equal to or greater than 60% with said sequence (i); from which at least one gene essential to assembly of said virus has been deleted; and
a nucleic acid sequence according to any of Claims 1-4.

8. Vector according to Claim 7, wherein said nucleic acid sequence according to any of Claims 1-4 has been inserted in a position of the nucleic acid sequence (I) other than its original position in the wild viral genome, or in a position adjacent to or immediately to the side of the deleted gene, or one of the deleted genes, in said nucleic acid sequence (I).

9. Vector according to Claim 7, wherein said nucleic acid sequence (I) is the complete genome sequence of a group 1 coronavirus, or a fragment thereof, from which at least one gene essential to the assembly of said coronavirus has been deleted, and in which said nucleic acid sequence according to any of Claims 1-4 has been located in a position other than its original position in the wild coronavirus.

10. Vector according to Claim 9, wherein said nucleic acid sequence (I) is the sequence of the complete genome of a group 1 coronavirus, or a fragment thereof, from which at least one gene essential to the assembly of said coronavirus has been deleted, and in which said nucleic acid sequence according to any of Claims 1-4 has been located in a position adjacent to or immediately to the side of the deleted gene or one of the deleted genes.

11. Vector according to any of Claims 7-10, which also includes a heterologous nucleic acid sequence which codes for a determined activity, under control of the transcription regulatory elements present in said vector.

12. Method for producing a product of interest that includes cultivating a host cell that contains a vector according to Claim 11 under conditions that permit the expression of the heterologous nucleic acid and recovery of the product of interest.

13. A method for producing a recombinant coronavirus that includes introducing a viral vector according to any of Claims 7-11 into a host cell, cultivating said host cell that contains said vector under conditions that permit the expression and replication of the vector and recovering the virions obtained from the recombinant coronavirus.

14. A host cell that includes a vector according to any of Claims 7-11.

15. A vaccine capable of protecting an animal against infection caused by an infectious agent that includes (i) at least one vector according to Claim 11, which expresses at least one antigen capable of inducing an immune response to said infectious agent, or an antibody that provides protection against said infectious agent, together with, optionally (ii) a pharmaceutically acceptable inactive ingredient.

16. Vaccine according to Claim 15, wherein said vector expresses at least one antigen capable of inducing a systemic immune response and/or an immune response in mucosa to different infectious agents that propagate in the respiratory or enteric mucosa.

17. A multivalent vaccine capable of protecting an animal against infection caused by more than one infectious agent including (i) a vector according to Claim 11, which expresses antigens capable of inducing an immune response to said infectious agents, or antibodies that provide protection against said infectious agents, together with, optionally (ii) a pharmaceutically acceptable inactive ingredient.

18. Multivalent vaccine capable of protecting an animal against infection caused by more than one infectious agent, including (i) more than one vector according to Claim 11, each of which express an antigen capable of inducing an immune response to each one of said infectious agents, or antibodies that provide protection against each one of said infectious agents, together with, optionally, (ii) a pharmaceutically acceptable inactive ingredient.


List of sequences
<110> BOARD OF SCIENTIFIC INQUIRY

<120> Nucleic acid sequence that includes the RNA encapsidation signal of a group 1
coronavirus and its applications

<160> 1

<170> PatentIn version 2.0

<210> 1
<211> 550
<212> cDNA
<213> Porcine transmissible gastroenteritis virus (TGEV)

<400> 1
GAAATATTTG TCTTTCTATG AAATCATAGA GGACAAGCGT TGATTATTTC CATTCAGTTT 60
GGCAATCACT CCTTGGAACG GGGTTGAGCG AACGGTGCAG TAGGGTTCCG TCCCTATTTC 120
GTAAGTCGCC TAGTAGTAGC GAGTGCGGTT CCGCCCGTAC AACGTTGGGT AGACCGGGTT 180
CCGTCCTGTG ATCTCCCTCG CCGGCCGCCA GGAGAATGAG TTCCAAACAA TTCAAGATCC 240
TTGTTAATGA GGACTATCAA GTCAACGTGC CTAGTCTTCC TATTCGTGAC GTGTTACAGG 300
AAATTAAGTA CTGCTACCGT AATGGATTTG AGGGCTATGT TTTCGTACCA GAATACTGTC 360
GTGACCTAGT TGATTGCGAT CGTAAGGATC ACTACGTCAT TGGTGTTCTT GGTAACGGAG 420
TAAGTGATCT TAAACCTGTT CTTCTTACCG AACCCTCCGT CATGTTGCAA GGCTTTATTG 480
TTAGAGCTAA CTGCAATGGC GTTCTTGAGG ACTTTGACCT TAAAATTGCT CGCACTGGCA 540
GAGGTGCCAT 550